(19) Europäisches Patentamt

European Patent Office

Office européen des brevets

(11) Publication number: **0 291 146 B1**

(12) **EUROPEAN PATENT SPECIFICATION**

(45) Date of publication of patent specification: **01.09.93**  (51) Int. Cl.⁵: **C12N 15/51**, C07K 3/12

(21) Application number: **88301779.0**

(22) Date of filing: **01.03.88**

(54) **Purification of recombinant hepatitis B surface antigen.**

(30) Priority: **09.03.87 US 23347**

(43) Date of publication of application:
**17.11.88 Bulletin 88/46**

(45) Publication of the grant of the patent:
**01.09.93 Bulletin 93/35**

(84) Designated Contracting States:
**AT BE CH DE ES FR GB IT LI LU NL SE**

(56) References cited:
**EP-A- 0 171 908**
**EP-A- 0 249 932**
**EP-A- 0 280 470**

(73) Proprietor: **MERCK & CO. INC.**
**126, East Lincoln Avenue P.O. Box 2000**
**Rahway New Jersey 07065-0900(US)**

(72) Inventor: **Hurni, William M.**
**329 Evergreen Drive**
**North Wales Pennsylvania 19454(US)**
Inventor: **Kubek, Dennis J.**
**1127 Vilsmeier Road**
**Lansdale Pennsylvania 19446(US)**
Inventor: **Miller, William J.**
**232 Old Church Road**
**North Wales Pennsylvania 19454(US)**
Inventor: **Rienstra, Mark S.**
**1519 Green Lane Road**
**Lansdale Pennsylvania 19446(US)**
Inventor: **Scolnick, Edward M.**
**811 Wickfield Road**
**Wynnewood Pennsylvania 19096(US)**

(74) Representative: **Cole, William Gwyn**
**European Patent Department, Merck & Co.,**
**Inc., Terlings Park, Eastwick Road**
**Harlow, Essex CM20 2OR (GB)**

Rank Xerox (UK) Business Services
(3.10/3.6/3.3.1)

## Description

### BACKGROUND OF THE INVENTION

In the production of recombinant hepatitis B surface antigen in yeast, a contaminating 60,000 dalton yeast protein associates with the hepatitis B surface antigen and is carried through the purification regimen of the surface antigen and appears in the final product. The contaminating yeast protein is closely associated with the surface antigen and cannot be removed from the 2,000,000 dalton surface antigen by conventional separation methods. The yeast impurity appears to be entrained within the interior of the surface antigen particle.

The presence of contaminating yeast protein in a vaccine product is undesirable.

### OBJECTS OF THE INVENTION

It is, accordingly, an object of the present invention to provide an improved method for purifying HBsAg produced by a transformed yeast. Another object is to provide a method for removing yeast impurities from HBsAg produced in transformed yeast. These and other objects of the present invention will become apparent from the following description.

### SUMMARY OF THE INVENTION

Yeast-derived proteins are removed from HBsAg produced in a transformed yeast by diafiltering the HBsAg with a dissociating agent across a membrane having a molecular weight cutoff greater than about 100,000 daltons but less than about 1,000,000 daltons, preferably from about 100,000 daltons to about 300,000 daltons.

### DETAILED DESCRIPTION

The present invention relates to a method of removing contaminating yeast protein from HBsAg produced in a transformed yeast.

Typically the process of the present invention involves the fermentation of a yeast that has been transformed with a vector coding for HBsAg.

The antigen thus obtained is then purified by conventional purification methods which are customarily used for the isolation and purification of biologically active substances, such as cell rupture, extraction of the ruptured cells, salting out with ammonium sulphate, gel filtration, ion exchange chromatography, fractionation with polyethyleneglycol, affinity chromatography, density gradient ultracentrifugation with sucrose and cesium chloride or sodium bromide, or the like.

Following conventional purification the HBsAg is treated according to the present invention to remove contaminating yeast protein. The treatment according to the present invention comprises diafiltering the desired HBsAg with a dissociating agent across a membrane having a molecular weight cutoff greater than about 50,000 daltons but less than about 1,000,000 daltons, preferably from about 100,000 daltons to about 300,000 daltons.

At the end of the fermentation, the yeast cells are broken to release the HBsAg and the cells and cell debris are removed by centrifugation. The supernatant liquid is then treated to remove waste proteins, for example by concentration and diafiltration. The liquid containing the product is then adsorbed to aerosil (fused silica) and washed to remove contaminating proteins. The aerosil is eluted with a suitable buffer and the HBsAg-containing material is concentrated, diafiltered and optionally centrifuged. The supernatant liquid from the centrifugation is passed through a butylagarose column and adsorbed antigen is eluted in a suitable buffer. The material is then concentrated and loaded onto a Sepharose® 6B column. Antigen-containing fractions from the Sepharose® column are pooled, diluted to a desired HBsAg protein concentration, and diluted with a dissociating agent, leading to dissociation of yeast protein from the hepatitis B protein. Because the yeast protein becomes entrained in the desired yeast-produced HBsAg particle, separation of the yeast protein is hindered and is not effected rapidly. Typically, the period of time to permit contaminating yeast proteins to dissociate is from about 6 hours to about 24 hours at room temperature although shorter times can be employed at higher temperatures. The mixture is then concentrated and diafiltered on a 100,000 molecular weight cutoff hollow fiber membrane using at least about five volumes of the dissociating agent as the diafiltration solution. The antigen is then diafiltered with at least about 15 volumes of phosphate buffered saline to remove the dissociating agent.

The transformed yeast can be prepared in known manner by introducing a gene encoding a desired polypeptide or protein into yeast cells which then express the encoded polypeptide or protein. For instance, the preparation of HBsAg antigen from the transformed yeast is described by Valenzuela et al., Nature 298:347 et seq. (1982). Another known method for preparing a yeast-origin HBsAg is described by Miyanohara et al., Proc. Natl. Acad. Sci., USA, 80:1 et seq. (1983).

The dissociating agent may be a polarity-reducing agent such as dioxane or ethylene glycol, urea, guanidine hydrochloride, or chaotropic agents such as $Cl^-$ $I^-$, $ClO^-_4$, $CF_3COO^-$, $SCN^-$, or $CCl_3COO^-$. Specific examples of suitable chaotropic agents are sodium perchlorate, sodium trifluoroacetate, sodium trichloroacetate, and alkali metal thiocyanates, e.g., NaSCN, KSCN, and NH₄SCN.

The following examples illustrate the present invention without, however, limiting the same thereto.

EXAMPLE 1

A recombinant hepatitis B surface antigen preparation consisting of 8 L of purified hepatitis B surface antigen in PBS contains approximately 100 µg/ml of protein by Lowry protein assay. Typically, 5 percent of the protein measured consists of a contaminating 60,000 dalton yeast protein. To the above solution, 8 L of 6M KSCN in PBS is added. The resulting 16 L of solution contains 50 µg/ml of protein and has a concentration of 3M KSCN. This solution is incubated with stirring for 16 hours at 4°C. At the end of the incubation period the solution is concentrated to 4 L in a 100 K molecular weight cutoff hollow fiber bundle (Amicon H10 P100-20). The resulting concentrate contains approximately 200 µg/ml protein in 3M KSCN. The hollow fiber bundle unit is then switched from concentration mode to diafiltration mode and the product is diafiltered using 20 L of 3M KSCN. The diafiltration mode removes the contaminating 60,000 dalton yeast protein that passes through the filter, from the 2,000,000 dalton hepatitis B surface antigen particle that is retained behind the filter. After completion of the diafiltration, the product is further diafiltered with 60 L of PBS to remove KSCN. The resulting product typically contains 100 µg/ml of protein in 6 L of which less than 1 percent of protein is yeast impurity as measured by HPLC.

EXAMPLE 2

A purified recombinant hepatitis B surface antigen containing Sepharose® 6B effluent is made 3M with respect to KSCN by adding solid KSCN. It is then stored overnight at 4°C. Two ml of this material is mixed with 8 ml of 3M KSCN in PBS and then concentrated to 2 ml using an Amicon minicon concentrator with an XM300 membrane having a size exclusion of greater than 300,000 daltons. Then 5 ml of 3M KSCN is added and the total volume again reduced to 2 ml by pressure filtration. Another 5 ml of 3M KSCN is added and the total volume is reduced to 1 ml. This one ml of product is then dialyzed against PBS overnight at room temperature to remove KSCN. The solutions before and after treatment were analyzed by polyacrylamide gel and immunoblot and the treated solution was found to be free of yeast protein while the untreated solution exhibited a band which was recognized by antibody to yeast impurity.

EXAMPLE 3

Purified recombinant hepatitis B surface antigen is made 3M with respect to KSCN. A second sample was then made 2.5M with respect to KSCN and a third sample was made 2M with respect to KSCN by addition in each case of solid KSCN. Following incubation each of these samples was diafiltered against 5 volumes of KSCN having the same molarity with respect to KSCN using either an XM300 (300,000 M.W. cutoff) membrane or an XM100 (100,000 M.W. cutoff) membrane. After the KSCN treatment, all of the samples were dialyzed against PBS overnight at room temperature to remove KSCN. All of the samples were analyzed by polyacrylamide gel and immunoblot. All conditions of membrane size and KSCN concentration removed the yeast impurity from the hepatitis B surface antigen.

**Claims**

1. A process for removing yeast-derived proteinaceous substances from a solution of hepatitis B surface antigen (HBsAg) particles produced in a transformed yeast which comprises diafiltering the contaminating yeast proteinaceous substance through a membrane having a molecular weight cutoff greater than about 100,000 daltons to about 1,000,000 daltons in the presence of a dissociating agent effective to dissociate the contaminating yeast proteinaceous substance from the HBsAg, the dissociating agent not being Triton® X-100.

2. A process according to Claim 1 wherein the dissociating agent is a polarity reducing agent or a chaotropic agent.

3. A process according to Claim 2 wherein the polarity reducing agent is dioxane, ethylene glycol, urea or guanidine hydrochloride.

4. A process according to Claim 2 wherein the chaotropic agent is $CCl_3COO^-$, $CF_3COO^-$. $Cl^-$, $ClO_4^-$, $I^-$ or $SCN^-$.

5. A process according to Claim 1 wherein the membrane has a molecular weight cutoff of from about 100,000 daltons to about 300,000 daltons.

6. A process according to Claim 1 wherein the solution of the HBsAg particles is incubated in the presence of the dissociating agent before diafiltering.

7. A process according to Claim 1 wherein the solution of the HBsAg particles is concentrated before diafiltering.

8. A process according to Claim 1 wherein the solution of the HBsAg particles is removed following diafiltering.

**Patentansprüche**

1. Verfahren zur Entfernung proteinartiger Substanzen, die von der Hefe abstammen, aus einer Lösung von Hepatitis B Oberflächenantigen (HBsAg)-Teilchen, hergestellt in einer transformierten Hefe, welches umfaßt Diafiltrieren der kontaminierenden proteinartigen Hefesubstanz durch eine Membran mit einer Molekulargewichtsgrenze von mehr als etwa 100 000 Dalton bis 1 000 000 Dalton in Gegenwart eines Dissoziationsmittels, welches wirksam ist, um die kontaminierende proteinartige Hefesubstanz von HBsAg zu dissoziieren, wobei das Dissoziationsmittel nicht Triton® X-100 ist.

2. Verfahren nach Anspruch 1, bei dem das Dissoziationsmittel ein polaritätsreduzierendes Mittel oder ein chaotropes Mittel ist.

3. Verfahren nach Anspruch 2, bei dem das polaritätsreduzierende Mittel Dioxan, Ethylenglycol, Harnstoff oder Guanidinhydrochlorid ist.

4. Verfahren nach Anspruch 2, bei dem das chaotrope Mittel $CCl_3COO^-$, $CF_3COO^-$, $Cl^-$, $ClO_4^-$, $I^-$ oder $SCN^-$ ist.

5. Verfahren nach Anspruch 1, bei dem die Membran eine Molekulargewichtsgrenze von etwa 100 000 Dalton bis etwa 300 000 Dalton aufweist.

6. Verfahren nach Anspruch 1, bei dem die Lösung der HBsAg-Teilchen vor dem Diafiltrieren in Gegenwart des Dissoziationsmittels inkubiert wird.

7. Verfahren nach Anspruch 1, bei dem die Lösung der HBsAg-Teilchen vor dem Diafiltrieren konzentriert wird.

8. Verfahren nach Anspruch 1, bei dem die Lösung der HBsAg-Teilchen nach dem Diafiltrieren entfernt wird.

**Revendications**

1. Procédé pour l'élimination de substances protéiques d'une solution de particules d'antigène de surface de l'hépatite B (AgsHB) produites dans une levure transformée, comprenant la diafiltration de la substance protéique contaminante de levure à travers une membrane ayant un seuil de coupure de masse moléculaire allant de plus d'environ 100 000 daltons à environ 1 000 000 daltons, en présence d'un agent de dissociation efficace pour dissocier la substance protéique contaminante de levure d'avec l'AgsHB, l'agent de dissociation n'étant pas le Triton® X-100.

2. Procédé selon la revendication 1, dans lequel l'agent de dissociation est un agent réducteur de polarité ou un agent chaotrope.

3. Procédé selon la revendication 2, dans lequel l'agent réducteur de polarité est le dioxanne, l'éthylène-glycol, l'urée ou le chlorhydrate de guanidine.

4. Procédé selon la revendication 2, dans lequel l'agent chaotrope est $CCl_3COO^-$, $CF_3COO^-$, $Cl^-$, $ClO_4^-$, $I^-$ ou $SCN^-$.

5. Procédé selon la revendication 1, dans lequel la membrane a un seuil de coupure de masse moléculaire allant d'environ 100 000 daltons à environ 300 000 daltons.

6. Procédé selon la revendication 1, dans lequel la solution des particules d'AgsHB est mise à incuber en présence de l'agent de dissociation, avant la diafiltration.

7. Procédé selon la revendication 1, dans lequel la solution des particules d'AgsHB est concentrée avant la diafiltration.

8. Procédé selon la revendication 1, dans lequel la solution des particules d'AgsHB est séparée après la diafiltration.